(19) Europäisches Patentamt
European Patent Office
Office européen des brevets

(11) **EP 3 527 989 B1**

(12) **EUROPEAN PATENT SPECIFICATION**

(45) Date of publication and mention
of the grant of the patent:
**25.11.2020 Bulletin 2020/48**

(51) Int Cl.:
**G01N 33/574** *(2006.01)*  **C07K 14/71** *(2006.01)*
**C12Q 1/68** *(2018.01)*

(21) Application number: **18382094.3**

(22) Date of filing: **18.02.2018**

(54) **METHOD, DEVICE AND KIT FOR THE EARLY DETECTION OF BREAST CANCER**

VERFAHREN, VORRICHTUNG UND KIT ZUR FRÜHERKENNUNG VON BRUSTKREBS

PROCÉDÉ, DISPOSITIF ET KIT POUR LA DÉTECTION PRÉCOCE DU CANCER DU SEIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**21.08.2019 Bulletin 2019/34**

(73) Proprietor: **Márquez Márquez, Lidia**
**28039 Madrid (ES)**

(72) Inventor: **Márquez Márquez, Lidia**
**28039 Madrid (ES)**

(74) Representative: **Sahuquillo Huerta, Jesús**
**Jesana IP**
**Apartado de Correos, 30**
**28300 Aranjuez (ES)**

(56) References cited:
- **BAYO J ET AL: "Analysis of blood markers for early breast cancer diagnosis", CLINICAL AND TRANSLATIONAL ONCOLOGY, SPRINGER ITALIA SRL, ITALY, SPAIN, vol. 20, no. 4, 14 August 2017 (2017-08-14), pages 467-475, XP036464965, ISSN: 1699-048X, DOI: 10.1007/S12094-017-1731-1 [retrieved on 2017-08-14]**
- **PEETER KARIHTALA ET AL: "Oxidative stress and counteracting mechanisms in hormone receptor positive, triple-negative and basal-like breast carcinomas", BMC CANCER, BIOMED CENTRAL, LONDON, GB, vol. 11, no. 1, 21 June 2011 (2011-06-21), page 262, XP021102374, ISSN: 1471-2407, DOI: 10.1186/1471-2407-11-262**

**Description**

**TECHNICAL FIELD OF THE INVENTION**

This invention refers to a method and device for the early detection of breast cancer and involves an analysis of a plurality of blood markers.

**PRIOR ART**

Breast cancer is a malignant proliferation of epithelial cells that line the mammary ducts and lobules. It is a clonal disease wherein an individual cell that is the product of a series of somatic or germline mutations acquires the capacity to divide itself without control or order, making it reproduce until it forms a tumour. This tumour, which starts as a slight anomaly, invades neighbouring tissue and finally spreads to other parts of the body. Therefore, an efficient and early diagnosis is necessary to prevent this possibility.

There are two main types of breast cancer. Infiltrating ductal carcinoma, which starts in the ducts that carry milk from the breast to the nipple is, by far, the most common - approximately 80% of cases-. In second place comes infiltrating lobular carcinoma - approximately 10% of cases which starts in the part of the breast called lobules, which produce breast milk. Taken as a whole, the remaining types of breast cancer do not exceed 10% of cases.

The main risk factors for contracting breast cancer include advanced age, first menstruation at a very early age, a first pregnancy at an advanced age or never having given birth, and family background. In between 5% to 10% of cases, breast cancer is caused by inherited genetic mutations.

Different tests are used to detect breast cancer such as the mammogram, mammary ultrasound with high-resolution transducers -sonography- an oestrogen and progesterone receptor test or magnetic resonance imaging. A definitive breast cancer diagnosis can only be determined by means of a breast biopsy.

In the prior art, breast cancer diagnosis methods by means of a blood analysis that detects antibodies compatible with the development of breast cancer, are described. These concern non-invasive tests -blood analysis- in which the serum is separated from the blood and, once separated, the antibodies found are analysed as described in documents EP2446272, WO9858978 and WO2008032084.

Another example described in the prior art is document US2015/0024960 which refers to early breast cancer diagnosis. More specifically, it refers to a group of biomarkers configured to diagnose the appearance of breast cancer in blood containing an antibody that recognises it specifically.

Notwithstanding, according to scientific consensus in this field, an efficient marker does not exist, nor does any predictive method contemplate its use. The prior art contemplates - exclusively- the use of markers CA 15.3 and CEA for monitoring the treatment of the advanced disease [Sturgeon C.M, Duffy M.J, Stenmam U-H, et al. National Academy of Clinical Biochemistry Laboratory Medicine Practice Guidelines for Use of Tumor Markers in Testicular, Prostate, Colorectal, Breast, and Ovarian Cancers. Clinical Chemistry (2008) Dec;54(12)] [Khatcheressian J.L, Hurley P. Bantug E, et al Breast Cancer Follow-Up and Management After PrimaryTreatment: American Society of Clinical Oncology Clinical Practice Guideline Update. J Clin Oncol 30. (2012)] y [Harris L, Fritsche H, Mennel R, et el. American Society of Clinical Oncology 2007 update of recommendations for the use of tumor markers in breast cancer. J Clin Oncol (2007); 25: 5287-312].

In the document [Bayo J, Castaño MA, Rivera F, Navarro F. Analysis of blood markers for early breast cancer diagnosis. Clin Transl Oncol. 2017 Aug 14] a series of markers for the early diagnosis of breast cancer have been published, which is considered to be the closest prior art to this invention. The purpose of this document and the study it describes is to determine, in patients diagnosed with cancer, the possible existence of any blood indicator that remains high and can therefore be used as a putative breast cancer predictive marker.

On the other hand, in this work markers are divided into three groups:

    i. A first group of markers which, from a clinical and scientific point of view are endorsed by a large number of works described in the prior art regarding breast cancer. In this first group are found the markers CEA and CA 15.3

    ii. A second group of markers which, although they do not have a clear relationship with breast cancer, are systematically used in clinical practice in the diagnosis of the disease such as, for example, CA 125, CYFRA 21.1, α-FETOPROTEIN, CA 19.9 and NSE (Neuron-Specific Enolase); and

    iii. a third group of experimental markers, such as NGAL, EGFR and 8-OHDG (respectively, NGAL (Neutrophil Gelatinase-Associated Lipocalin), EGFR (Epidermal Growth Factor Receptor) and 8-OHDG (8-hydroxy-2'-deoxyguanosine)) which have been studied in some breast cancer series but not for the specific purpose of early diagnosis.

In the document [Bayo J, Castaño MA, Rivera F, Navarro F. Analysis of blood markers for early breast cancer diagnosis. Clin Transl Oncol. 2017 Aug 14]an analytical observational epidemiological study has been designed with case design and controls, which includes 63 cases and 63 controls. The cases are patients diagnosed with localised breast cancer (cT1-2 and cN0) waiting to be operated on. The selection criteria for these patients were: (a) having been diagnosed with operable breast cancer; (b) not having previously suffered another tumour; (c) that the disease is not in an advanced or metastatic phase; (d) had not previously received neoadjuvant cancer treatment; and (e) accepts being included in the study.

With respect to the control group, it is characterised as healthy women whose selection criteria were that they were not suffering chronic pathologies or had any history of cancer. They also had to accept, logically, being included in the study.

With respect to statistical analysis for both quantitative and descriptive characteristics; proportional comparisons based on the chi-square test and means comparison based on Student's t-test, were used to tackle the main objective. Then a binary logistic regression analysis using the Wald method was performed, then ROC curve methodology was undertaken to finish off.

The two series turned out to be similar. Notwithstanding, the control series had a lower average age (45 years compared to 57 years) and, therefore, a lower proportion of menopause and, furthermore, a higher employment rate compared to the group of housewives. The cases series, however, had lower levels of vitamin D and higher BMI (body mass index). The rest of the characteristics are well balanced.

The description of the results of [Bayo J, Castaño MA, Rivera F, Navarro F. Analysis of blood markers for early breast cancer diagnosis. Clin Transl Oncol. 2017 Aug 14] can be summarised, firstly, that the analysis of the markers reflected significant differences in both groups for six markers: four routine (CYFRA, NSE, CEA AND CA 15.3) and two experimental (EGFR and 8-OHDG). However, when the cut-off points in markers with a normal range were applied, only CA 15.3 proved to be significant. Sensitivity was very low (11%) and so its usefulness individually in early diagnosis was ruled out.

The EGFR was significantly higher in the controls, as indicated in previous prior art publications. However, the 8-OHDG marker was significantly higher in the cases, this being the first time this marker has been studied in early breast cancer diagnosis. Furthermore, by means of logistic regression analysis and the construction of a ROC curve, a mathematical equation made up of five markers was obtained, i.e. CA 15.3, NSE, NGAL, EGFR and 8-OHDG which achieve a correct breast cancer diagnosis probability of 91.8%.

## EXPLANATION OF THE INVENTION

The object of this invention is a method, a device and a kit for the early diagnosis of breast cancer which, starting from the scientific findings of the experimentation undertaken in the document [Bayo J, Castaño MA, Rivera F, Navarro F. Analysis of blood markers for early breast cancer diagnosis. Clin Transl Oncol. 2017 Aug 14], improve the efficiency of the diagnosis and increase its simplicity.

Another object of this invention is to increase the success rate of classic breast cancer diagnosis markers. This object is reached with the method of claim 1. In dependent claims, the use of additional markers that increase the effectiveness of the invention method is described.

The invention method, the device this method uses and the diagnostic kit helps support different clinical cases by means of the inclusion of a calculation algorithm which, through a simple blood extraction, enables a plurality of tumour markers, the presence or absence of the disease, to be determined with a success rate higher than 90%.

The method, device and kit described in this invention can be used in large high-risk groups of the population of different ages to supplement or replace mammograms, being a non-invasive method, does not induce iatrogenic radiations and, therefore, can be repeated as often as necessary, and, furthermore, is suitable for all ages.

Finally, it is worth indicating, for those skilled in the art, that other objects, benefits and characteristics of the invention will emanate from the description, drawings and claims. Furthermore, the invention covers all possible combinations of particular and preferred embodiments indicated here.

## BRIEF DESCRIPTION OF THE DRAWINGS

Here below is a very brief description of a series of drawings that help to understand the invention better and which expressly relates to an embodiment of said invention which is illustrated by way of a non-limiting example of it.

FIG.1 shows a ROC curve of a first example of the practical embodiment of the invention.
FIG.2 shows a second ROC curve of a second example of the practical embodiment of the invention.
FIG.3 shows a third ROC curve of a third example of the practical embodiment of the invention.

## EXPLANATION OF A DETAILED EMBODIMENT OF THE INVENTION

Starting from the work undertaken and described in [Bayo J, Castaño MA, Rivera F, Navarro F. Analysis of blood markers for early breast cancer diagnosis. Clin Transl Oncol. 2017 Aug 14] the trial described has been repeated and all the possible correlations have been analysed, having surprisingly identified that the inverse behaviour of the 8-OHDG and EGFR markers enables its quotient to be evaluated in the early diagnosis of breast cancer. The invention method enables the clinical utility of the 8-OHDG/EFGR quotient to be determined along with other tumour markers in the initial phase of breast cancer.

With regard to the analyses obtained by means of the trial described in [Bayo J, Castaño MA, Rivera F, Navarro F. Analysis of blood markers for early breast cancer diagnosis. Clin Transl Oncol. 2017 Aug 14] a new cross-sectional descriptive study was undertaken with 62 patients with localised breast cancer waiting for surgical intervention and 62 healthy women.

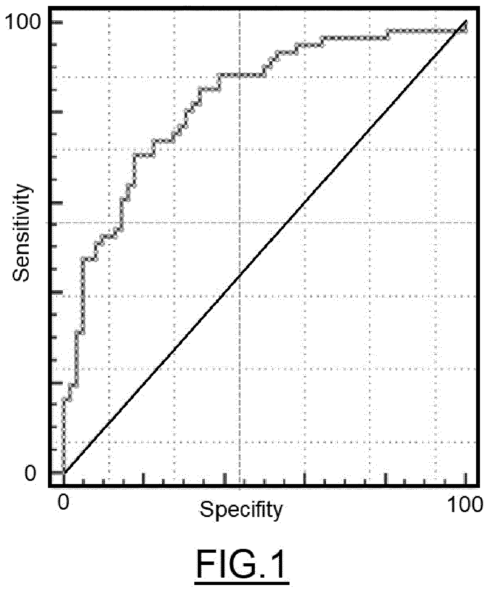

Once the CA 15.3, CEA, CA125, CA 19.9, NSE, CYFRA 21.1, $\alpha$-FETOPROTEIN and experimental breast cancer markers (NGAL, EGFR y 8-OHDG) have been determined, defining the ratio (8-OHDG)/(EGFR)*100. To compare the levels by groups, the Mann-Whitney U test and multivariate logistic regression (henceforth, of *Wald*) is used to predict the probability of cancer according to the 8-OHDG/EGFR ratio, in isolation and with the rest of the markers evaluated. The diagnostic performance has been evaluated by means of the ROC curve area (figures 1 to 3).

Higher levels of EGFR were detected in the controls (5.097 versus 5.81 ng/ml with p<0.001) and 8OHDG in the cases (9.85 versus 7.37 ng/ml with p<0.001) differences that are more marked with the 8OHDG/ EFGR ratio (198 versus 122 with p<0.001).

The CEA, CYFRA, NSE and NGAL markers were higher in the patients (p<0.05) although their behaviour in isolation showed scant diagnostic sensitivity. Furthermore, factors such as age, menopause, job, BMI and low levels of vitamin D proved to be risk variables for the disease. Logistic regression analysis of the ratio evaluated yielded a performance in isolation of 82.4% (see example 1) which rose to 91.2% (see example 2) by combining said quotient with other markers, obtaining a multivariate predictive equation that includes NSE and NGAL, which can improve to up to 92.8% owing to the synergistic interaction of different markers (see example 3).

Example 1. Clinical interest of the 8-OHDG/EGFR ratio. Bivariate analysis.

The tables below show the results of the analyses of the trial results for early breast cancer diagnosis. Figure 1 shows the ROC curve of the trial.

Mann-Whitney Test (for independent samples):

|  | Sample 1 (Cases) | Sample 2 (Controls) |
|---|---|---|
| Size of the sample | 62 | 62 |
| Lowest value | 0.0465 | 0.377 |
| Highest value | 9.462 | 2.920 |
| Median | 1.976 | 1.219 |
| 95% CIfor the median | 1.776 to 2.270 | 1.068 to 1.393 |
| Interquartile range | 1.527 to 2.554 | 0.022 to 1.547 |

| | |
|---|---|
| Average range of sample 1 | 82.098 |
| Average range of sample2 | 42.226 |
| Mann-Whitney U | 665.00 |
| Z statistic (larger sample) | 6.202 |
| Probability | p<0.0001 |

Logistic regression and ROC curve area of figure 1:

| | |
|---|---|
| Area of ROC curve | 0.824 |
| Standard error | 0.038 |

(continued)

| 95% confidence interval | 0.750 to 0.898 |
|---|---|
| Z statistic | 8.548 |
| Probability | p<0.0001 |

Thus, the logistic regression analysis of the ratio evaluated yielded a performance of 82.4% with a p<0.0001for the use of the 8OHDG/EGFR ratio in isolation in the early diagnosis of breast cancer at a significantly lower cost than the method proposed in [Bayo J, Castaño MA, Rivera F, Navarro F. Analysis of blood markers for early breast cancer diagnosis. Clin Transl Oncol. 2017 Aug 14].

Example 2. Clinical interest of the 8-OHDG/EGFR ratio. Multivariate analysis.

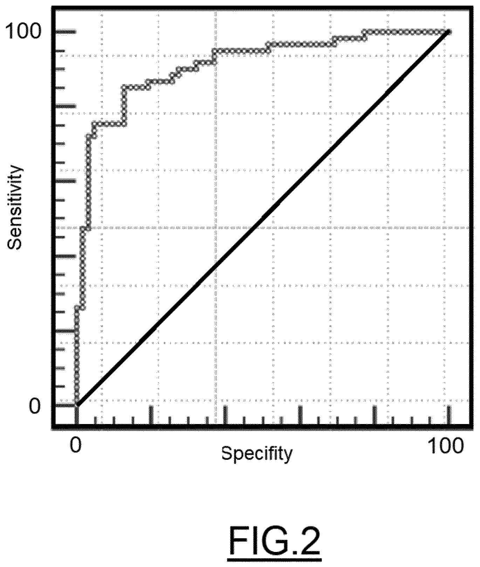

The tables below show the results of the analyses of the trial results for early breast cancer diagnosis. Figure 2 shows the ROC curve of the trial. In this example the predictive value improves to 91.2% in a non-iterative model, with the use of the 8OHDG/EGFR ratio and the NSE and NGAL markers, in the early detection of breast cancer at a significantly lower cost than the method proposed in the document [Bayo J, Castaño MA, Rivera F, Navarro F. Analysis of blood markers for early breast cancer diagnosis. Clin Transl Oncol. 2017 Aug. 14]as it uses one marker (CA 15.3) less than the aforementioned analysis.

Estimated parameters by means of multivariate logistic regression:

| Iter.1 | B | E.T. | WALD | GL | SIGMA | EXP(B) |
|---|---|---|---|---|---|---|
| 8OHDG/EGFR | 2.588 | 0.650 | 21.378 | 1 | 0.000 | 13.304 |
| NSE | -0.096 | 0.030 | 10.030 | 1 | 0.002 | 0.908 |
| CA153 | 0.061 | 0.039 | 2.455 | 1 | 0.117 | 1.063 |
| CA125 | 0.039 | 0.042 | 0.877 | 1 | 0.349 | 1.040 |
| NGAL | -0.303 | 0.128 | 5.582 | 1 | 0.018 | 0.739 |
| CYFRA | 0.626 | 0.436 | 2.066 | 1 | 0.151 | 1.871 |
| CEA | 0.188 | 0.155 | 1.483 | 1 | 0.223 | 1.207 |
| Constant | -4.752 | 1.332 | 12.730 | 1 | 0.000 | 0.009 |

Where B is the estimated parameter, ET is the typical error, GL are the degrees of freedom and SIGMA is the standard deviation. The three parameters with the lowest standard deviations will be taken into account (8OHDG/EGFR, NSE and NGAL ratio).

The summary of the model is as follows:

| Step | -2log-likelihood | $R^2$of Cox and Snell | $R^2$Nagelkerke |
|---|---|---|---|
| 1 | 93.807 | 0.464 | 0.619 |

In this case it should be taken into account that the estimate finished at iteration number 6 because the estimates of the parameters changed by less than 0.001. Thus, the formula is as follows:

$$Logit = -4.752 + 2.588 * \left(\frac{8 - OHDG}{EGFR}\right) - 0.096 * (NSE) - 0.303(NGAL)$$

$$P = \frac{e^{Logit}}{1 + e^{Logit}} * 100$$

Logistic regression area and ROC curve of figure 2:

| Area of the ROC curve | 0.912 |
|---|---|
| Standard error | 0.027 |
| 95% confidence interval | 0.859 to 0.966 |
| Z statistic | 15.187 |
| Probability | p<0.0001 |

Thus, the logistic regression analysis of the ratio evaluated yielded a performance of 91.2% with a p<0.0001.

Example 3. Clinical interest of the 8-OHDG/EGFR ratio. Multivariate analysis.

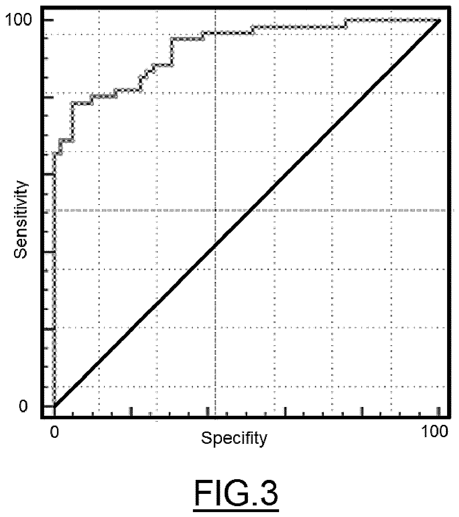

The tables below show the results of the analyses of the trial results for early breast cancer diagnosis. Figure 3 shows the ROC curve of the trial. In this example the predictive value improves to 92.8% in a non-iterative model, with the use of the 8OHDG/EGFR ratio and the NSE, NGAL and NGAL*CA15.3 markers, in the early detection of breast cancer with greater diagnostic precision.

Below are shown the estimated parameters by means of multivariate logistic regression in iteration 11, which offers the best result of the logistic regression calculation:

| Iter.11 | B | E.T. | WALD | GL | SIGMA | EXP(B) |
|---|---|---|---|---|---|---|
| 8OHDG/EGFR | 5.125 | 1.053 | 23.697 | 1 | 0.000 | 168.168 |
| NSE | -0.282 | 0.088 | 10.376 | 1 | 0.001 | 0.754 |
| RATIONGAL | -0.471 | 0.123 | 14.724 | 1 | 0.000 | 0.624 |
| CYFRANSE | 0.140 | 0.054 | 6.655 | 1 | 0.010 | 1.151 |
| NGALCA153 | 0.023 | 0.008 | 7.855 | 1 | 0.005 | 1.023 |
| Constant | -5.634 | 1.346 | 17.527 | 1 | 0.000 | 0.004 |

Where B is the estimated parameter, ET is the typical error, GL are the degrees of freedom and SIGMA is the standard deviation. With regard to the parameters, they are RATIO (i.e. the relationship between 8OHDG/EGFR), NSE (marker NSE), RATIONGAL (i.e. the product between RATIO, which is the relationship between the markers 8OHDG/EGFR, and the marker NGAL), CYFRANSE (i.e. the relationship between the markers CYFRA and NSE) and NGALCA153 (i.e. the relationship between the markers NGAL and CA 15.3).

The three parameters with the lowest standard deviations will be considered (without taking CYFRANSE into account). Thus, the formula is as follows:

$$Logit = -5.634 + 5.125 * RATIO\left(\frac{8-OHDG}{EGFR}\right) - 0.282 * (NSE) - 0.471 * RATIO\left(\frac{8-OHDG}{EGFR}\right)$$
$$* (NGAL) + 0.4 * (NGAL) * (CA153)$$

$$P = \frac{e^{Logit}}{1 + e^{Logit}} * 100$$

Area of the logistic regression and ROC curve of figure 3:

| Area of the ROC curve | 0.928 |
|---|---|
| Standard error | 0.025 |
| 95% confidence interval | 0.879 to 0.976 |
| Z statistic | 17.292 |

(continued)

| Probability | p<0.0001 |
| --- | --- |

Thus, the logistic regression analysis of the ratio evaluated yielded a performance of 92.8% with a p<0.0001.

It should be noted that, during the trials, it was shown that interaction exists between some markers and the equation with interaction (example 3) and without interaction between them (example 2) has been trialled, and it was observed that, taking into account the interactions (synergistic relationships) between markers, the results with respect to prediction are better (92.8% compared to 91.2%), a fact that was not described in the prior art.

The invention method can be implemented in different ways. Thus, for example, it can be implemented in a device comprising means configured to execute the invention method or can be distributed by means of a kit comprising the markers indicated in any of the embodiments (examples 1 to 3) and means to execute the method described which, logically, comprises means to undertake blood or urine analysis as well as means to calculate the probability of breast cancer affectation according to the examples described and which can be easily implemented, for example, in an IT system with sufficient calculation capacity.

This IT system, in a non-limiting way, can be anything from an application executable on a computer, tablet or mobile phone to a dedicated electronic device, the only required condition being that it implements the formulae indicated in each one of the examples, by means of instructions executable by a processor.

**Claims**

1. A method for the early detection of breast cancer comprising: (a) detecting and quantifying the presence of markers 8-OHDG, NSE, NGAL, CA 15.3 and EGFR in blood or urine; (b) establishing the probability of breast cancer affectation according to a logistic regression of the values detected and quantified in blood or urine of a combination of the markers 8-OHDG, EGFR, NSE, CA 15.3 and NGAL; and wherein the calculus of the probability of breast cancer affectation comprises: (i) the calculation of the ratio of the values detected and quantified of the marker 8-OHDG with respect to the values detected and quantified in of the marker EGFR; (ii) the product of the ratio (8-OHDG/EGFR) in step (i) by the value detected and quantified of the marker NGAL; and (iii) the calculation of the product of the values detected and quantified of the markers NGAL and CA 15.3; and wherein the logistic regression of the values detected and quantified in blood or urine of a combination of the markers 8-OHDG, EGFR, NSE, CA 15.3 and NGAL is calculated according with the following formula:

$$Logit = -5.634 + 5.125 * RATIO\left(\frac{8-OHDG}{EGFR}\right) - 0.282 * (NSE) - 0.471 * RATIO\left(\frac{8-OHDG}{EGFR}\right) * (NGAL) + 0.4 * (NGAL) * (CA153)$$

$$P = \frac{e^{Logit}}{1 + e^{Logit}} * 100$$

2. A device for the early detection of breast cancer comprising a processor or processors arranged to execute a plurality of instructions to calculate the probability of breast cancer affectation according to a logistic regression of the values detected and quantified in blood or urine of a combination of the markers 8-OHDG, EGFR, NSE, CA 15.3 and NGAL; and wherein the calculus of the probability of breast cancer affectation comprises: (i) the calculation of the ratio of the values detected and quantified of the marker 8-OHDG with respect to the values detected and quantified in of the marker EGFR; (ii) the product of the ratio (8-OHDG/EGFR) in step (i) by the value detected and quantified of the marker NGAL; and (iii) the calculation of the product of the values detected and quantified of the markers NGAL and CA 15.3; and wherein the logistic regression of the values detected and quantified in blood or urine of a combination of the markers 8-OHDG, EGFR, NSE, CA 15.3 and NGAL is calculated according with the following formula:

$$Logit = -5.634 + 5.125 * RATIO\left(\frac{8-OHDG}{EGFR}\right) - 0.282 * (NSE) - 0.471 * RATIO\left(\frac{8-OHDG}{EGFR}\right) * (NGAL) + 0.4 * (NGAL) * (CA153)$$

$$P = \frac{e^{Logit}}{1 + e^{Logit}} * 100$$

3. A software product configured to be executed by a processor or processors comprising a plurality of instructions to calculate the probability of breast cancer affectations according to a logistic regression of the values detected and quantified in blood or urine of a combination of the markers 8-OHDG, EGFR, NSE, CA 15.3 and NGAL; and wherein the calculus of the probability of breast cancer affectation comprises: (i) the calculation of the ratio of the values detected and quantified of the marker 8-OHDG with respect to the values detected and quantified in of the marker EGFR; (ii) the product of the ratio (8-OHDG/EGFR) in step (i) by the value detected and quantified of the marker NGAL; and (iii) the calculation of the product of the values detected and quantified of the markers NGAL and CA 15.3; and wherein the logistic regression of the values detected and quantified in blood or urine of a combination of the markers 8-OHDG, EGFR, NSE, CA 15.3 and NGAL is calculated according with the following formula:

$$Logit = -5.634 + 5.125 * RATIO\left(\frac{8-OHDG}{EGFR}\right) - 0.282 * (NSE) - 0.471 * RATIO\left(\frac{8-OHDG}{EGFR}\right) * (NGAL) + 0.4 * (NGAL) * (CA153)$$

$$P = \frac{e^{Logit}}{1 + e^{Logit}} * 100$$

**Patentansprüche**

1. Verfahren zur Früherkennung von Brustkrebs, umfassend: (a) Nachweis und Quantifizierung des Vorhandenseins der Marker 8-OHDG, NSE, NGAL, CA 15.3 und EGFR in Blut oder Urin; (b) Ermittlung der Wahrscheinlichkeit einer Brustkrebsbeeinträchtigung gemäß einer logistischen Regression der in Blut oder Urin einer Kombination der Marker 8-OHDG, EGFR, NSE, CA 15.3 und NGAL nachgewiesenen und quantifizierten Werte; und wobei der Kalkül der Wahrscheinlichkeit einer Brustkrebsbeeinträchtigung umfasst: (i) die Berechnung des Verhältnisses der vom Marker 8-OHDG detektierten und quantifizierten Werte zu den in dem Marker EGFR detektierten und quantifizierten Werten; (ii) das Produkt des Verhältnisses (8-OHDG / EGFR) in Schritt (i) durch den vom Marker NGAL detektierten und quantifizierten Wert; und (iii) die Berechnung des Produkts der erfassten und quantifizierten Werte der Marker NGAL und CA 15.3; und wobei die logistische Regression der in Blut oder Urin nachgewiesenen und quantifizierten Werte einer Kombination der Marker 8-OHDG, EGFR, NSE, CA 15.3 und NGAL gemäß der folgenden Formel berechnet wird:

$$Logit = -5.634 + 5.125 * RATIO\left(\frac{8-OHDG}{EGFR}\right) - 0.282 * (NSE) - 0.471 * RATIO\left(\frac{8-OHDG}{EGFR}\right) * (NGAL) + 0.4 * (NGAL) * (CA153)$$

$$P = \frac{e^{Logit}}{1 + e^{Logit}} * 100$$

2. Vorrichtung zur Früherkennung von Brustkrebs, umfassend einen Prozessor oder Prozessoren, die angeordnet sind, um eine Vielzahl von Anweisungen auszuführen, um die Wahrscheinlichkeit einer Brustkrebsbeeinträchtigung gemäß einer logistischen Regression der in Blut oder Urin einer Kombination erfassten und quantifizierten Werte zu berechnen der Marker 8-OHDG, EGFR, NSE, CA 15.3 und NGAL; und wobei der Kalkül der Wahrscheinlichkeit einer Brustkrebsbeeinträchtigung umfasst: (i) die Berechnung des Verhältnisses der vom Marker 8-OHDG erfassten und quantifizierten Werte zu den in dem Marker EGFR nachgewiesenen und quantifizierten Werten; (ii) das Produkt des Verhältnisses (8-OHDG / EGFR) in Schritt (i) durch den vom Marker NGAL detektierten und quantifizierten Wert; und (iii) die Berechnung des Produkts der erfassten und quantifizierten Werte der Marker NGAL und CA 15.3; und wobei die logistische Regression der in Blut oder Urin nachgewiesenen und quantifizierten Werte einer Kombination der Marker 8-OHDG, EGFR, NSE, CA 15.3 und NGAL gemäß der folgenden Formel berechnet wird:

$$Logit = -5.634 + 5.125 * RATIO\left(\frac{8-OHDG}{EGFR}\right) - 0.282 * (NSE) - 0.471 * RATIO\left(\frac{8-OHDG}{EGFR}\right)$$
$$* (NGAL) + 0.4 * (NGAL) * (CA153)$$

$$P = \frac{e^{Logit}}{1 + e^{Logit}} * 100$$

**3.** Ein Softwareprodukt, das konfiguriert ist, um von einem Prozessor oder Prozessoren ausgeführt zu werden, das eine Vielzahl von Anweisungen umfasst, um die Wahrscheinlichkeit von Brustkrebserkrankungen gemäß einer logistischen Regression der in Blut oder Urin einer Kombination der Marker 8 erfassten und quantifizierten Werte zu berechnen -OHDG, EGFR, NSE, CA 15.3 und NGAL; und wobei der Kalkül der Wahrscheinlichkeit einer Brustkrebsbeeinträchtigung umfasst: (i) die Berechnung des Verhältnisses der vom Marker 8-OHDG erfassten und quantifizierten Werte zu den in dem Marker EGFR nachgewiesenen und quantifizierten Werten; (ii) das Produkt des Verhältnisses (8-OHDG / EGFR) in Schritt (i) durch den vom Marker NGAL detektierten und quantifizierten Wert; und (iii) die Berechnung des Produkts der erfassten und quantifizierten Werte der Marker NGAL und CA 15.3; und wobei die logistische Regression der in Blut oder Urin nachgewiesenen und quantifizierten Werte einer Kombination der Marker 8-OHDG, EGFR, NSE, CA 15.3 und NGAL gemäß der folgenden Formel berechnet wird:

$$Logit = -5.634 + 5.125 * RATIO\left(\frac{8-OHDG}{EGFR}\right) - 0.282 * (NSE) - 0.471 * RATIO\left(\frac{8-OHDG}{EGFR}\right)$$
$$* (NGAL) + 0.4 * (NGAL) * (CA153)$$

$$P = \frac{e^{Logit}}{1 + e^{Logit}} * 100$$

**Revendications**

**1.** Procédé de détection précoce du cancer du sein comprenant: (a) la détection et la quantification de la présence de marqueurs 8-OHDG, NSE, NGAL, CA 15.3 et EGFR dans le sang ou l'urine; (b) établir la probabilité d'affectation du cancer du sein selon une régression logistique des valeurs détectées et quantifiées dans le sang ou l'urine d'une combinaison des marqueurs 8-OHDG, EGFR, NSE, CA 15.3 et NGAL; et dans lequel le calcul de la probabilité d'affectation du cancer du sein comprend: (i) le calcul du rapport des valeurs détectées et quantifiées du marqueur 8-OHDG par rapport aux valeurs détectées et quantifiées dans le marqueur EGFR; (ii) le produit du rapport (8-OHDG / EGFR) à l'étape (i) par la valeur détectée et quantifiée du marqueur NGAL; et (iii) le calcul du produit des valeurs détectées et quantifiées des marqueurs NGAL et CA 15.3; et dans lequel la régression logistique des valeurs détectées et quantifiées dans le sang ou l'urine d'une combinaison des marqueurs 8-OHDG, EGFR, NSE, CA 15.3 et NGAL est calculée selon la formule suivante:

$$Logit = -5.634 + 5.125 * RATIO\left(\frac{8-OHDG}{EGFR}\right) - 0.282 * (NSE) - 0.471 * RATIO\left(\frac{8-OHDG}{EGFR}\right)$$
$$* (NGAL) + 0.4 * (NGAL) * (CA153)$$

$$P = \frac{e^{Logit}}{1 + e^{Logit}} * 100$$

2. Un dispositif pour la détection précoce du cancer du sein comprenant un processeur ou des processeurs agencés pour exécuter une pluralité d'instructions pour calculer la probabilité d'affectation du cancer du sein selon une régression logistique des valeurs détectées et quantifiées dans le sang ou l'urine d'une combinaison des marqueurs 8-OHDG, EGFR, NSE, CA 15.3 et NGAL; et dans lequel le calcul de la probabilité d'affectation du cancer du sein comprend: (i) le calcul du rapport des valeurs détectées et quantifiées du marqueur 8-OHDG par rapport aux valeurs détectées et quantifiées dans le marqueur EGFR; (ii) le produit du rapport (8-OHDG / EGFR) à l'étape (i) par la valeur détectée et quantifiée du marqueur NGAL; et (iii) le calcul du produit des valeurs détectées et quantifiées des marqueurs NGAL et CA 15.3; et dans lequel la régression logistique des valeurs détectées et quantifiées dans le sang ou l'urine d'une combinaison des marqueurs 8-OHDG, EGFR, NSE, CA 15.3 et NGAL est calculée selon la formule suivante:

$$Logit = -5.634 + 5.125 * RATIO\left(\frac{8 - OHDG}{EGFR}\right) - 0.282 * (NSE) - 0.471 * RATIO\left(\frac{8 - OHDG}{EGFR}\right)$$
$$* (NGAL) + 0.4 * (NGAL) * (CA153)$$

$$P = \frac{e^{Logit}}{1 + e^{Logit}} * 100$$

3. Un produit logiciel configuré pour être exécuté par un ou plusieurs processeurs comprenant une pluralité d'instructions pour calculer la probabilité d'affectations du cancer du sein selon une régression logistique des valeurs détectées et quantifiées dans le sang ou l'urine d'une combinaison des marqueurs 8 -OHDG, EGFR, NSE, CA 15.3 et NGAL; et dans lequel le calcul de la probabilité d'affectation du cancer du sein comprend: (i) le calcul du rapport des valeurs détectées et quantifiées du marqueur 8-OHDG par rapport aux valeurs détectées et quantifiées dans le marqueur EGFR; (ii) le produit du rapport (8-OHDG / EGFR) à l'étape (i) par la valeur détectée et quantifiée du marqueur NGAL; et (iii) le calcul du produit des valeurs détectées et quantifiées des marqueurs NGAL et CA 15.3; et dans lequel la régression logistique des valeurs détectées et quantifiées dans le sang ou l'urine d'une combinaison des marqueurs 8-OHDG, EGFR, NSE, CA 15.3 et NGAL est calculée selon la formule suivante:

$$Logit = -5.634 + 5.125 * RATIO\left(\frac{8 - OHDG}{EGFR}\right) - 0.282 * (NSE) - 0.471 * RATIO\left(\frac{8 - OHDG}{EGFR}\right)$$
$$* (NGAL) + 0.4 * (NGAL) * (CA153)$$

$$P = \frac{e^{Logit}}{1 + e^{Logit}} * 100$$

## FIG.1

FIG.2

## FIG.3

## REFERENCES CITED IN THE DESCRIPTION

### Patent documents cited in the description

- EP 2446272 A
- WO 9858978 A
- WO 2008032084 A
- US 20150024960 A

### Non-patent literature cited in the description

- **STURGEON C.M ; DUFFY M.J ; STENMAM U-H et al.** National Academy of Clinical Biochemistry Laboratory Medicine Practice Guidelines for Use of Tumor Markers in Testicular, Prostate, Colorectal, Breast, and Ovarian Cancers. *Clinical Chemistry,* December 2008, vol. 54 (12
- **KHATCHERESSIAN J.L ; HURLEY P. BANTUG E et al.** Breast Cancer Follow-Up and Management After PrimaryTreatment: American Society of Clinical Oncology Clinical Practice Guideline Update. *J Clin Oncol,* 2012, vol. 30
- **HARRIS L ; FRITSCHE H ; MENNEL R.** American Society of Clinical Oncology 2007 update of recommendations for the use of tumor markers in breast cancer. *J Clin Oncol,* 2007, vol. 25, 5287-312
- **BAYO J ; CASTAÑO MA ; RIVERA F ; NAVARRO F.** Analysis of blood markers for early breast cancer diagnosis. *Clin Transl Oncol,* 14 August 2017
- **BAYO J ; CASTAÑO MA ; RIVERA F ; NAVARRO F.** Analysis of blood markers for early breast cancer diagnosis. *Clin Transl Oncol.,* 14 August 2017